Europäisches Patentamt

European Patent Office

Office européen des brevets

(11) Veröffentlichungsnummer: **0 301 292 B1**

(12) **EUROPÄISCHE PATENTSCHRIFT**

(45) Veröffentlichungstag der Patentschrift: **11.12.91**

(51) Int. Cl.⁵: **A61F 6/02, A61F 2/48**

(21) Anmeldenummer: **88110928.4**

(22) Anmeldetag: **08.07.88**

(54) Verschluss für eine männliche Harnröhre.

(30) Priorität: 28.07.87 DE 3724875
27.06.88 DE 3821631

(43) Veröffentlichungstag der Anmeldung:
01.02.89 Patentblatt 89/05

(45) Bekanntmachung des Hinweises auf die
Patenterteilung:
11.12.91 Patentblatt 91/50

(84) Benannte Vertragsstaaten:
AT BE CH DE ES FR GB GR IT LI LU NL SE

(56) Entgegenhaltungen:
DE-A- 2 431 888
DE-C- 504 554
DE-C- 602 099
US-A- 4 183 358
US-A- 4 555 242

(73) Patentinhaber: **Bader, Paul**
**Strutweg 1**
**W-7070 Schwäbisch Gmünd(DE)**

(72) Erfinder: **Bader, Paul**
**Strutweg 1**
**W-7070 Schwäbisch Gmünd(DE)**

**Beschreibung**

Die Erfindung betrifft einen Verschluss für eine männliche Harnröhre, der von einem in die Harnröhre einzuführenden und darin zu befestigenden länglichen Körper gebildet ist.

Das Verschließen der männlichen Harnröhre kann aus verschiedenen Gründen notwendig sein. Einmal kann es zum Schutz des Bettes gegen Bettnässen oder Pollution angezeigt sein. In einem anderen Fall soll durch den Verschluß der Harnröhre das Austreten des Spermas beim Gechlechtsverkehr als Mittel zur Geburtenregelung verhindert werden.

Aus DE-OS 1 566 405 ist eine Vorrichtung zur sicheren und unschädlichen Familienplanung bekannt, die dadurch gekennzeichnet ist, daß ein in die männliche Harnröhre einzuführender kurzer, schlanker, an einem Ende geschlossener Schlauch aus gut dehnbaren Weichgummi am anderen Ende luft- und wasserdicht mit dem Halsteil eines Füllröhrchens verbunden ist, das die Fortsetzung des Ventilkopfes bildet. Mittels einer Spritze oder einem Druckball wird Wasser oder Luft in den Schlauch eingepresst, so daß dieser sich dehnt, wodurch die Vorrichtung in der Harnröhre festgehalten wird.

In der DE-OS 1 957 693 wird eine Vorrichtung zur Familienplanung vorgeschlagen, die dadurch gekennzeichnet ist, daß in einem, in die männliche Harnröhre einzuführenden kurzen, schlanken, dehnbaren Schlauch aus Weichgummi o.dgl., der an seinem offenen Ende fest mit dem Halsteil der Vorrichtung verbunden ist, eine Anzahl von Spreizhebeln angeordnet sind, die durch Drehung einer im Kopfteil angeordneten Schraube soweit gespreizt werden, daß der Schlauch so gedehnt wird, daß er sich mit seinem am stärksten gedehnten Ende, dicht an den inneren Rand des collum glandis angelegt und damit einerseits einen vollkommen dichten Verschluss der Harnröhre bewirkt und gleichzeitig einen festen Halt in diesem Organ gewährleistet.

Laut den Ausführungen des Erfinders der beiden vorzitierten Erfindungen - einem Mediziner - sollen die anatomischen Verhältnisse beim Mann zur Anbringung eines Schwangerschaftsverhütungsmittels "geradezu ideal" sein. Und trotzdem wird mit Ausnahme des Präservativs "die Masse der üblichen Schwangerschaftsverhütungsmittel" im Körper der Frau vorgenommen.

Die Vorrichtung, die im DE-OS 1 957 693 vorgeschlagen wird, hat aber den entscheidenden Nachteil, daß diese nicht dicht ist und die Samenflüssigkeit zwischen der Harnröhrenwand und dem Verschluss austreten kann. Bei der Dehnung des Schlauches mittels einer Anzahl kleiner Spreizhebel, wird ein Polygon gebildet. Dadurch werden zwischen den Anlegepunkten zwei parallele Flächen gebildet, die nicht dichten können, da die Zugkräfte mit denen die beiden Flächen gespannt sind, unendlich groß sein müßten.

Die wesentlichen Nachteile, die beide vorgenannte Erfindungen jedoch gemeinsam haben, ist, daß der aus dem männlichen Glied herausragende Ventilkopf sich in jeder Beziehung störend auswirkt, sei es beim Tragen der Vorrichtung oder beim Ausüben des Geschlechtsverkehrs. Außerdem muß die Vorrichtung zum Wasserlassen jedesmal aus der Harnröhre entfernt und anschließend wieder eingeführt werden, wenn diese Vorrichtung von Inkontinenzkranken getragen wird.

Aus der US-A-4.183.358 ist eine Vorrichtung zur Empfängnisverhütung für den Mann bekannt, die aus einem Harnröhrenverschluss gebildet ist. Dieser ist von einem länglichen Körper 18 gebildet, der in die Harnröhre eingeführt und darin befestigt ist. Dieser längliche Körper 18 ist im wesentlichen zylindrisch ausgebildet und an beiden Enden offen. An seinem vorderen Ende weist der Körper ein scheibenförmiges Teil 24 auf, das die Eichel des Gliedes wenigstens im Bereich der Harnröhrenöffnung ummantelt. In dem länglichen Körper 18 ist an seinem vorderen Ende ein Verschluß 30 angeordnet.

Nachteilig bei dieser Art von Harnröhrenverschluss ist, daß sowohl der Verschluss, als auch der längliche Körper 18 über den Penis hinaussteht und sich beim Geschlechtsakt störend auswirkt. Desweiteren wird die Eichel von einem scheibenförmigen Teil 24, das den länglichen Körper bildet, gerade im nervlich empfindlichsten Teil abgedeckt, so daß auch dadurch empfindsame Beeinträchtigungen im Geschlechtsakt auftreten. Außerdem ist nicht auszuschließen, daß die über die Eichel hinausragenden Teile des Harnröhrenverschlusses Schmerzen bei der Frau hervorrufen.

Diese Vorrichtung ist darüberhinaus zum Einsatz beim Inkontinenzkranken nicht geeignet.

In der DE-A-2431888 und in der US-A4.555.242 sind zwei Abdichtungen für Verschlüsse künstlicher Körperöffnungen beschrieben. Die Abdichtung erfolgt dabei durch eine, in der Körperöffnung angeordnete, die Vorrichtung umschließende einfache Membrane, wobei diese Membrane im aufgeblähten Zustand die Bauchhaut im Bereich der Körperöffnung gegen eine dort, auf die Außenseite der Bauchhaut breitflächig angeordnete Auflageplatte 2 (nach DE-24 31 888) verpreßt.

Diese Art der Dichtung ist für eine Abdichtung der Harnröhre nicht anwendbar. Die Harnröhre würde übermäßig gedehnt und dadurch Schmerzen hervorrufen. Die Auflageplatte 2 würde das Reizzentrum der empfindlichen Eichel des Penis abdecken und unempfindsam machen. Beim Einsatz als Urinsperre bei Inkontinenzkranken müßte zum Wasserlassen der Verschluss jedesmal aus der

Harnröhre entfernt und anschließend wieder eingesetzt werden.

Die DE-C-602 099 beschreibt eine Vorrichtung zur Beseitigung der impotentia coeundi. Dabei wird ein dünnes Weichgummirohr, das von einer, bis zu einem bestimmten Grad, ausdehnbaren Hülle umgeben ist,in die Harnröhre eingeführt. Die aus gummiertem, beschränkt dehnbaren Gewebe bestehende Hülle von entsprechender Länge ist an ihrem offenen Ende fest mit einem, am unteren Ende geschlossenen, abgerundeten, dünnen Rohr aus Weichgummi, welches zahlreiche seitliche Öffnungen aufweist, verbunden. Die Hülle liegt in kontrahiertem Zustand dem Rohr in seiner ganzen Länge dicht und glatt an. Das offene Ende des Rohres mündet in einen Weichgummikopf. In dem Weichgummikopf befindet sich eine Bohrung mit einem Rückschlagventil. An dem Weichgummikopf ist außen eine verstärkte Gummihülle befestigt, die an ihrem freien Ende in einem kräftigen Weichgummiring ausläuft.

Mit dem unteren Ende wird das Weichgummirohr mit der Hülle in die Harnröhre eingeführt. Hierauf wird die Gummihülle über die Eichel gezogen, so daß ein Gummiring in den sulcus coronaris des Penis zu liegen kommt. Hierdurch wird die Vorrichtung in der richtigen Lage gehalten. Dann wird mit einer kleinen Pumpe Luft oder Wasser durch die Bohrung mit dem Rückschlagventil im Weichgummikopf in das Weichgummirohr eingepreßt, wobei die Luft oder das Wasser durch die seitlichen Öffnungen in die Hülle austritt und diese soweit ausdehnt, daß sie die nötige Form und Festigkeit erlangt, um die Errektion des Gliedes zu ermöglichen. Gleichzeitig wird mit Hilfe des Weichgummirings und der inneren Kompression ein kombinierter Druck in der Gegend des sulcus coronaris ausgeübt, so daß der Rückfluß des Blutes aus den Schwellkörpern der Eichel gehemmt und dadurch eine entsprechende Vergrößerung des Organs erzielt wird.

Nachteilig an dieser Erfindung ist der Weichgummikopf mit der verstärkten Gummihülle, die über die Eichel gezogen wird. Beide, sowohl Weichgummikopf als auch die verstärkte Gummihülle decken den sensiblen Bereich der Eichel ab und machen diese mehr oder weniger unempfindsam. Darüberhinaus wird der den Penis überragenden Weichgummikopf als störend beim Geschlechtsverkehr empfunden werden.

Es ist die Aufgabe der vorliegenden Erfindung, eine Vorrichtung zum Verschluss der männlichen Harnröhre so zu verbessern, daß diese absolut flüssigkeitsdicht in der Harnröhre anordenbar ist und diese auf verhältnismäßig großer Fläche außen abdichtet und daß zum Wasserlassen diese Vorrichtung nicht aus der Harnröhre entfernt zu werden braucht und wobei diese Vorrichtung nach kurzer Eingewöhnungsphase kein störendes Empfinden hervorruft und wobei das Einführen und das Entfernen leicht und ohne große Umstände jederzeit durchführbar ist und daß die Vorrichtung kostengünstig herstellbar ist. Eine weitere Aufgabe diese Erfindung ist es, den Verschluss so zu gestalten, daß eine männliches Glied zusätzlich künstlich steif gehalten wird.

Diese Aufgabe wird erfindungsgemäß dadurch gelöst, daß der längliche Körper von einem Grundkörper oder von einem Grundkörper mit wenigstens einem Zwischenglied und einem Endglied gebildet ist und so einen ein- oder mehrgliedrigen, beidseitig offenen zylindrischen Ventilkörper mit durchgehender Längsbohrung bildet, wobei der Ventilkörper wenigstens an seinem vorderen Ende einen lösbar angeordneten Verschluss aufweist und wobei der Ventilkörpermantel teilweise von einem druckbeaufschlagbaren Doppelmembran-Schlauch ummantel ist, daß der Doppelmembran-Schlauch von einer inneren, am Ventilkörper glatt anliegenden und einer äußeren, dehnbaren Membrane gebildet ist und daß der Grundkörper im vorderen Bereich wenigstens eine radiale Bohrung aufweist, und daß die innere Mantelmembrane im Bereich der radialen Bohrung eine Luftdurchtrittsöffnung aufweist.

Ein zylindrischer Ventilkörper läßt sich einfach und umkompliziert in die Harnröhre einführen. Die Wand des zylindrischen Ventilkörpers legt sich über ihre ganze Mantelfläche gleichmäßig an der Harnröhrenwand an. So wird die Voraussetzung für eine gute flächige Abdichtung geschaffen.

Der Ventilkörper ist mit einer durchgehenden Längsbohrung ausgestattet und ebenfalls an seinem vorderen und hinteren Ende offen, wenn der lösbare Verschuss aus dem Ventilkörper herausgenommen ist. Dadurch ist es möglich "Wasserzulassen" ohne den Ventilkörper aus der Harnröhre vorher herauszunehmen und hinterher wieder einzuführen.

Der lose Verschluss schließt im eingebauten Zustand den Ventilkörper völlig luft- und flüssigkeitsfest, so daß dieser absolut sicher gegen Bettnässen und Spermaaustritt ist.

Der druckbeaufschlagte Doppelmembram-Schlauch mit einer inneren, am Ventilkörper glatt anliegenden Membrane der den Ventilkörpermantel dicht umschließt und einer äußeren dehnbaren Membrane hält den Ventilkörper absolut dicht und unverrutschbar in der Harnröhre fest.

Der mehrgliedrige Ventilkörper ist zusätzlich für ein künstliches Steifhalten des männlichen Gliedes einsetzbar.

In vorteilhafter Weiterbildung können bei einem mehrgliedrigen Ventilkörper der Grundkörper, das wenigstens eine Zwischenglied und das Endglied paßgenau und beweglich unlösbar ineinander eingepaßt sein. Außerdem können der Grundkörper, das oder die Zwischenglieder und das Endglied gleiche Innen- und Außendurchmesser aufweisen. Gleichzeitig können der eingliedrige Ventilkörper und das Endglied des mehrgliedrigen Ventilkörpers am Mantel ihres jeweiligen hinteren Ende eine Abrundung aufweisen.

Durch die Verwendung eines mehrgliedrigen Ventilkörpers, der aus einem Grundkörper, wenigstens einem Zwischenglied und einem Endglied zusammengesetzt ist, ergibt sich automatisch die weitere Funktion für eine zusätzliche künstliche Versteifung des männlichen Gliedes. Dabei können der Grundkörper, das oder die Zwischenglieder und das Endglied so ineinander eingepaßt sein, daß die Verbindungen unlösbar, aber in Grenzen bewegbar, sind. Durch die bewegliche Anordnung der einzelnen Glieder untereinander, kann sich der "verlängerte Verschluß" einerseits leicht der natürlichen Krümmung des männlichen Gliedes anpassen und andererseits ist das Glied immer noch beweglich genug, um insbesondere beim Geschlechtsverkehr keine Schmerzen zu verursachen.

Dadurch, daß alle Glieder gleiche Innen- und Außendurchmesser aufweisen, ergeben sich weder Probleme beim Einschieben des Verschlusses in die Harnröhre noch treten irgendwelche Ablaufschwierigkeiten beim "Wasserlassen" auf. Durch die Abrundung am hinteren Ende des eingliedrigen Ventilkörpers und am Endglied des mehrgliedrigen Ventilkörpers können diese ohne Schwierigkeiten und ohne Schmerzen zu verursachen in die Harnröhre eingeführt werden.

Durch den gleichen Außendurchmesser am vorderen Ende, wie am hinteren Ende des Harnröhrenverschlusses kann der Ventilkörper mit seinem vorderen Ende soweit in die Harnröhre eingeschoben werden, daß dieseshinter der Harnröhrenöffnung angeordnet ist, so daß kein störendes Teil, z.B. ein Ventilkopf aus der Harnröhre herausragt. Ein Herausholen des Verschlusses aus der Harnröhre kann entweder durch Zug am Faden erfolgen oder man schiebt den ganzen Verschluß mit Hilfe der Finger von hinten aus der Harnröhre heraus, dadurch, daß der oder die Finger durch leichten Druck auf den Penis den Ventilkörper von hinten aus der Harnröhre herausdrücken.

In Ausgestaltung vorstehender Ausführungen kann bei einem mehrgliedrigem Ventilkörper im Bereich der Einpaßstellen vom Grundkörper, Zwischen- und Endglied in der durchgehenden Längsbohrung im Ventilkörper eine Innenbeschichtung angeordnet sein. Diese Innenbeschichtung ist flexibel und kann die Bewegungen der Glieder mitmachen, ohne dabei beschädigt zu werden. Die Innenbeschichtung hat die Aufgabe die Einpaßstellen flüssigkeitsdicht zu beschichten, sodaß keine Harnröhrenflüssigkeit in die Einpasse eindringen kann. Es soll damit erreicht werden, daß keinen Nester entstehen können, die irgendwelche Infektionsquellen darstellen könnten.

Von Vorteil kann es sein, wenn im Grundkörper des Ventilkörpers an dessen vorderen Ende eine beidseitig offene zylindrische Büchse angeordnet ist. In dieser Büchse kann als Verschluß ein herausnehmbarer Stopfen angeordnet sein, wobei die Außenkante der Büchse und die Außenfläche des Stopfens bündig mit der Vorderkante des Ventilkörpers abschließen.

Die beidseitig offene Büchse, die am vorderen Ende des Grundkörpers des Ventilkörpers angeordnet ist, hat die Aufgabe, die in diesen Bereich des Grundkörpers angeordnete radiale Bohrung zu verschließen, so daß kein Harn in die radiale Bohrung eintreten kann. Der Stopfen, der in die Büchse hineingedrückt ist und mit der Vorderkante des Ventilkörpers bündig abschließt, verschließt die innere Längsbohrung flüssigkeitsdicht, so daß beim Verkehr keine Samenflüssigkeit austreten kann. Ein Faden, der an dem Stopfen eingearbeite ist und ringförmig aus dem Stopfen herausragt, dient dazu, den Stopfen aus der Büchse herauszuziehen (beim Wasserlassen). Die Büchse kann mit Hilfe eine Mini-Abzugsbolzens leicht aus dem Grundkörper entfernt werden.

Die äußere Membrane kann entweder glatt oder als Faltenbalg oder lamellenbalgartig ausgebildet sein. Die innere Membrane kann glatt am Ventilkörper anliegen und im Bereich der radialen Bohrung im Grundkörper einen Verstärkerpilzkopf aufweisen.

Die Ausgestaltung der äußeren Membrane in entweder glatt oder als Faltenbalg oder als Lamellenbalg stellt eine spezifische Ausgestaltungsform der äußeren Membrane dar. Die innere Membrane muß immer glatt sein und am Ventilkörper bündig anliegen. Der Verstärkerpilzkopf auf der inneren Membrane dient als Rückschlagventil. Er soll verhindern, daß Gas oder Flüssigkeit, die in den Hohlraum des Doppelmembran- Schlauches zwecks Verspannung des Ventilkörpers in der Harnröhre eingebracht worden ist, austreten kann. Das Pressgas oder die Pressflüssigkeit wird, mittels eines Gummibalges, über eine Ventil durch die radiale Bohrung im Ventilkörper, in den Doppelmembran-Schlauch eingepumpt und zwar durch die Luftdurchtrittsöffnung in der Mantelmembrane, die in geringer Entfernung hinter der radialen Bohrung vorgesehen ist.
Der Abstand zwischen der radialen Bohrung und der Luftdurchtrittsöffnung ist bewußt so gewählt, weil die innere Membrane und die äußere Membrane etwa im Bereich der Eichel des Penis zusammengeklebt sind, so daß sich in diesem Bereich die äußere Membrane nicht ausdehnen kann, was Schmerzen verursachen würde.

Mit der vorgeschlagenen Erfindung läßt sich ein sicherer Schutz des Bettes gegen Bettnässen und gegen Pollution schaffen. Da die Erfindung sich auch hervorragend zur Empfängnisverhütung eignet,
wird wahrscheinlich ein großer Teil der Produktion auf diesen Marktsektor absetzbar sein. Vergleicht man die handelsüblichen Produkte auf dem Markt der Empfängnisverhütung, so weist die vorgeschlagene Erfindung denen gegenüber doch erhebliche Vorteile auf:

Die vorgeschlagene Erfindung hat bei sachgemäßer Behandlung eine sehr hohe Lebensdauer. Lediglich die Membrane und evtl. auch der Dichtteil, also der Verstärkerpilzkopf sind gelegentlich erneuerungsbedürftig. Für die Membrane dürfte die Lebensdauer bei etwa einem Jahr liegen. Die Verschlußeinrichtung der Stopfen dürfte ca. 5 - 10 Jahre halten. Die Erneuerung dürfte keine Schwierigkeiten bereiten.

Von der Hygiene aus betrachtet, gibt es heute kein vergleichbares Mittel.

Vom Empfinden her betrachtet, macht die vorgeschlagene Erfindung den Mann eher männlich, weshalb auch eine verlängerte Form alternativ vorgeschlagen wird. Die vorgeschlagene Erfindung läßt sich als Massenprodukt in Großserien herstellen. Sie ist somit kostengünstig.

Vorteilhafte Weiterbildungen und erfinderische Ausgestaltungen ergeben sich aus dem in folgendem, anhand der Zeichnung prinzipmäßig dargestellten Ausführungsbeispiel.

Es zeigen:

Figur 1: Die erfindungsgemäße Vorrichtung eines mehrgliedrigen Ventilkörpers in Seitenansicht im Schnitt in vergrößerter Darstellung, wobei die Einzelglieder einzeln hintereinander gezeichnet dargestellt sind.

Figur 2: Einen Ausschnitt aus Figur 1 mit dem Verschluss am vorderen Ende des Grundkörpers in Seitenansicht im Schnitt.

Figur 3: Ein Schnittbild eines eingliedrigen Ventilkörpers in Seitenansicht.

Figur 4: Einen Ausschnitt aus der alternativen Verschlussmöglichkeit am vorderen Ende des Grundkörpers in Seitenansicht im Schnitt.

Figur 5: Ein Schnittbild eines Aufblasbalges mit dem Einlaßventil und einem Dorn.

Ein mehrgliedriger Ventilkörper 4 ist von einem Grundkörper 1 von einem Zwischenglied 2 und einem Endglied 3 gebildet. Ein eingliedriger Ventilkörper 5 besteht aus einem Grundkörper 1.

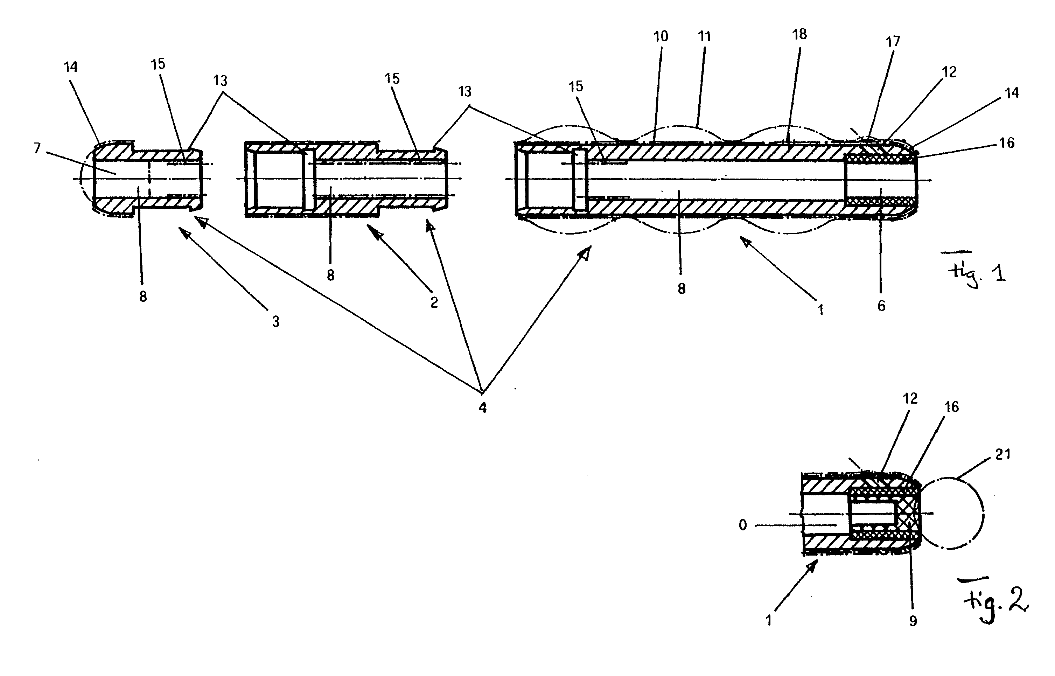

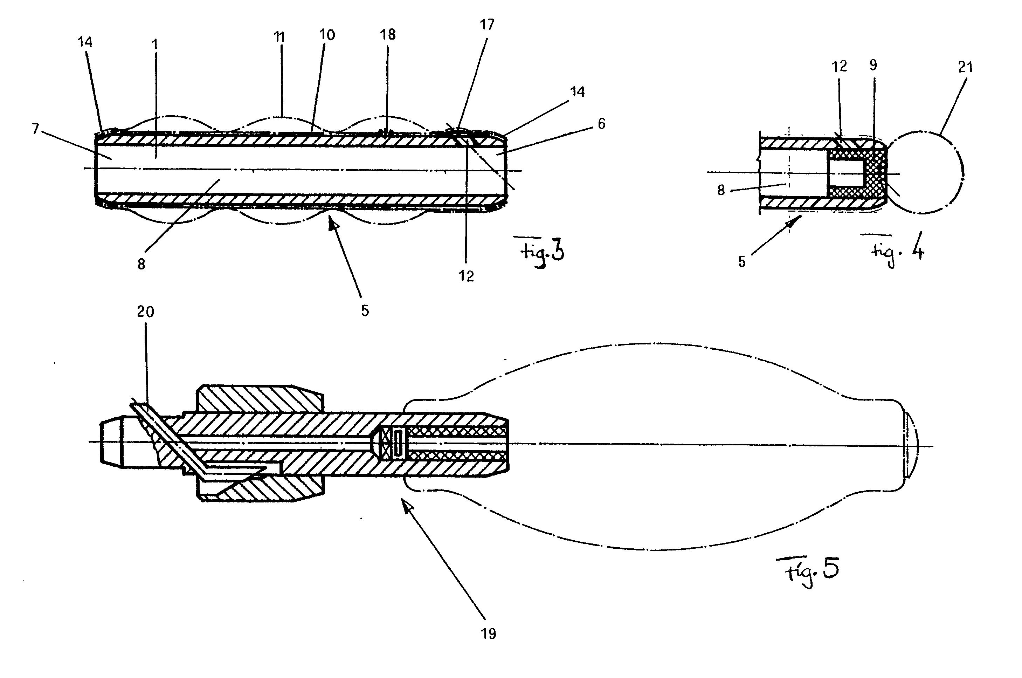

Wie aus den Figuren 1 und 3 ersichtlich ist, sind der mehrgliedrige Ventilkörper 4 und der eingliedrige Ventilkörper 5 zylindrisch ausgebildet. Sie sind an den Enden mit einer vorderen Öffnung 6 und einer hinteren Öffnung 7 offen und besitzen eine durchgehende Längsbohrung 8, die die vordere Öffnung 6 und die hintere Öffnung 7 miteinander verbindet. An seinem vorderen Ende weist sowohl der mehrgliedrige Ventilkörper 4, als auch der eingliedrige Ventilkörper 5 einen jeweils in der vorderen Öffnung 6 angeordneten Verschluss 9 in Form eines Stopfens auf (Figur 2 und Figur 4). Der Ventilkörper 4 und 5 ist an seinem Mantel wenigstens teilweise von einem Doppelmembran-Schlauch 10,11 umschlungen. An ihren vorderen Enden weisen sowohl der eingliedrige Ventilkörper 5, als auch der mehrgliedrige Ventilkörper 4 je eine radiale Bohrung 12 auf.

Bei dem mehrgliedrigen Ventilkörper 4 sind der Grundkörper 1, das Zwischenglied 2 und das Endglied 3 paßgenau über den Einpaß 13 unlösbar ineinandergepaßt. Am Einpaß 13 sind die einzelnen Glieder zueinander beweglich. Der Grundkörper 1, das Zwischenglied 2 und das Endglied 3 weisen gleichen Innen- und Außendurchmesser auf. Sowohl der eingliedrige Ventilkörper 5, als auch der mehrgliedrige Ventilkörper 4 weisen an ihrem jeweiligen hinteren Ende eine Abrundung 14 auf. Bei deM mehrgliedrigen Ventilkörper 4 nach Figur 1 ist im Bereich der Einpaßstellen 13 vom Grundkörper 1 zum Zwischenglied 2 und vom Zwischenglied 2 zum Endglied 3 in der durchgehenden Längsbohrung 8 eine Innenbeschichtung 15 angeordnet.

Im Grundkörper 1 des Ventilkörpers 4 (Figur 1) ist an dessen vorderen Ende eine beidseitig offene zylindrische Büchse 16 angeordnet, in der als Verschluss 9 ein herausnehmbarer Stopfen angeordnet ist. Am Stopfen befindet sich ein Faden 21. Der Verschluß 9, kann wie in Figur 4 dargestellt, auch direkt - also ohne Zwischenbüchse - in das vordere Ende des Grundkörpers 1 eingesetzt werden.

Die Außenkante der Büchse 16, die Außenfläche des Verschlusses 9 und die Vorderkante des Ventilkörpers 4 oder 5 sind bündig.

Der Membran-Doppelschlauch 10,11 ist von einer inneren Mantelmembrane 10 und einer äußeren Membrane 11 gebildet. Die Mantelmembrane 10 ist nur in sehr engen Grenzen dehnbar und umschließt den Ventilkörper 4 und 5 eng, insbesondere im Bereich des Grundkörpers 1. Dabei überdeckt die innere Mantelmembrane 10 auch die radiale Bohrung 12. Zur Verstärkung ist an der Mantelmembrane 10 im Bereich der radialen Bohrung 12 ein Verstärkerpilzkopf 17 vorgesehen, weil dort die Membrane durch den mechanischen Dorn sehr stark belastet ist. Eine Luftaustrittsöffnung 18 ist in unmittelbarer Nähe der radialen Bohrung 12 in der Mantelmembrane 10 vorgesehen.

Den zweiten Teil des Mantelmembran-Schlauches 10,11 bildet die äußere Membrane 11. Sie umschließt die Mantelmembrane 10 und ist dehnbar. Die Mantelmembrane 10 ist vorzugsweise nur im Bereich des Grundkörpers 1 angeordnet. Im vorderen Bereich des Grundkörpers 1 beginnt der dehnbare, aufblasbare Teil der äußeren Membrane 11 etwa 3 cm hinter der Vorderkante des Grundkörpers 1 und endet etwa am Ende des Grundkörpers 1.

Das Anordnen des Verschlusses in einer männlichen Harnröhre ist einfach. Der ein- oder mehrgliedrige Ventilkörper 4 oder 5 wird in die Harnröhre eingeführt und zwar soweit, bis der Grundkörper 1 ganz in der Harnröhre verschwunden ist. Anschließend wird mittels einer kleinen Handpumpe 19, die in die vordere Öffnung 6 eingeschoben wird, Luft oder Flüssigkeit durch die radiale Bohrung 12 in die äußere Membrane 11 gedrückt. Dabei hebt ein Dorn 20 die mit dem Verstärkerpilzkopf versehene Mantelmembrane leicht an, um die Luft- oder Flüssigkeitsfüllung zu erleichtern. Die Luft- oder die Flüssigkeit wandert bis zur Luftdurchtrittsöffnung 18 zwischen der Oberfläche des Grundkörpers 1 und der Außenhaut der Mantelmembrane 10. Durch die Luft bzw. Flüssigkeit wird die äußere Membrane aufgebläht und gegen die Harnröhrenwand gedrückt und befestigt so den Grundkörper 1 des Ventilkörpers 4 oder 5 in der Harnröhre. Gleichzeitig presst der Innendruck im Doppelmembran-Schlauch 10,11 den Verstärkerpilzkopf 17 mit der in diesem Bereich angeordneten Mantelmembrane 10 auf die radiale Bohrung 12 und dichtet diese ab.

Beim Geschlechtsverkehr ist der Verschluss 9 fest in dem Grundkörper 1 angeordnet. Zum Wasserlassen kann der Verschluss 9 aus dem Grundkörper 1 entfernt und danach wieder eingesetzt werden.

Zum Entfernen des Ventilkörpers 4 oder 5 aus der Harnröhre wird mit einem kleinen Stab oder dem Dorn 20 der Handpumpe 19 die Membrane 10 mit dem Verstärkerpilzkopf 17 durch die radiale Bohrung 12 angehoben, so daß das Druckmedium aus dem Zwischenraum des Doppelmembran-Schlauches 10, 11 entweichen kann. Anschließend wird der Verschluß aus der Harnröhre herausgezogen.

Selbstverständlich besteht der gesamte Ventilkörper aus hautfreundlichem Material. Er ist somit körperverträglich.

**Patentansprüche**

1. Verschluss für eine männliche Harnröhre, der von einem in die Harnröhre einzuführenden und darin zu befestigenden länglichen Körper gebildet ist,
**dadurch gekennzeichnet,**
daß der längliche Körper von einem Grundkörper (1) oder von einem Grundkörper (1) mit wenigstens einem Zwischenglied (2) und einem Endglied (3) gebildet ist und so einen ein- oder mehrgliedrigen, beidseitig offenen zylindrischen Ventilkörper (4 und 5) mit durchgehender Längsbohrung (8) bildet, wobei der Ventilkörper (4 und 5) wenigstens an seinem vorderen Ende einen lösbar angeordneten Verschluss (9) aufweist und wobei der Ventilkörpermantel teilweise von einem druckbeaufschlagbaren Doppelmembran-Schlauch (10, 11) ummantelt ist, daß der Doppelmembran-Schlauch (10, 11) von einer inneren Mantelmembrane (10), die am Ventilkörper (4 und 5) glatt anliegt und einer äußeren, dehnbaren Membrane (11) gebildet ist und daß der Grundkörper (1) im vorderen Bereich wenigstens eine radiale Bohrung (12) aufweist, und daß die innere Mantelmembrane im Bereich der radialen Bohrung (12) eine Luftdurchtrittsöffnung (18) aufweist.

2. Verschluss für eine männliche Harnröhre nach Anspruch 1
**dadurch gekennzeichnet,**
daß bei einem mehrgliedrigen Ventilkörper (4) der Grundkörper (1), das wenigstens eine Zwischenglied (2) und das Endglied (3) paßgenau und beweglich unlösbar ineinander eingepaßt sind und daß Grundkörper (1), Zwischenglied (2) und Endglied (3) gleichen Innen- und Außendurchmesser aufweisen.

3. Verschluss für eine männliche Harnröhre nach einem der Ansprüche 1 oder 2,
**dadurch gekennzeichnet,**
daß bei einem mehrgliedrigen Ventilkörper (4) im Bereich der Einpaßstellen (13) vom Grundkörper (1), Zwischenglied (2) und Endglied (3) in der durchgehenden Längsbohrung (8) im mehrgliedrigen Ventilkörper (4) einen Innenbeschichtung (15) angeordnet ist.

4. Verschluss für eine männliche Harnröhre nach einem der Ansprüche 1 bis 3,
**dadurch gekennzeichnet,**
daß der eingliedrige Ventilkörper (5) und das Endglied (3) des mehrgliedrigen Ventilkörpers (4) am Mantel ihres jeweiligen hinteren Endes eine Abrundung (14) aufweisen.

5. Verschluss für eine männliche Harnröhre nach einem der Ansprüche 1 bis 4,
**dadurch gekennzeichnet,**
daß im Grundkörper (1) des Ventilkörpers (4; 5) an dessen vorderen Ende eine beidseitig offene zylindrische Büchse (16) angeordnet ist, in der als Verschluss (9) ein herausnehmbarer Stopfen angeordnet ist, wobei die Außenkante der Büchse (16) und die Außenkante des Verschlusses (9) bündig mit der Vorderkante des Ventilkörpers (4; 5) sind.

6. Verschluss für eine männliche Harnröhre nach Anspruch 1,
**dadurch gekennzeichnet,**
daß die äußere Membrane (11) entweder glatt oder als Faltenbalg oder als Lamellenbalg ausgebildet ist.

7. Verschluss für eine männliche Harnröhre nach einem der Ansprüche 1 bis 6,
**dadurch gekennzeichnet,**
daß die innere Mantelmembrane (10) im Bereich der radialen Bohrung (12) im Grundkörper (1) einen Verstärkerpilzkopf (17) aufweist.

**Claims**

1. A seal for a male urethra, which is formed from an elongate body to be introduced into the urethra and secured therein, characterised in that the elongate body is formed from a base body (1) or from a base body (1) with at least one intermediate element (2) and an end element (3), and thus forms a one-section or multi-section cylindrical valve body (4 and 5), open at both sides, with a continuous longitudinal bore (8), wherein the valve body (4 an 5) has at least on its front end a detachably arranged seal (9) and wherein the valve body jacket is encased partially by a double membrane tube (10,11), acted upon by pressure, in that the double membrane tube (10,11) is formed by an internal jacket membrane (10), which lies evenly against the valve body (4 and 5) and an external extensible membrane (11), and in that the base body (1) has in the front region at least one radial bore (12), and in that the internal jacket membrane has in the region of the radial bore (12) an air-passage opening (18).

2. A seal for a male urethra according to claim 1, characterised in that with a multi-section valve body (4) the base body (1), at least one intermediate element (2) and the end element (3) are fitted into each other in an exact manner and in a movably non-detachable manner and in that the base body (1), intermediate element (2) and end element (3) have the same internal and external diameters.

3. A seal for a male urethra according to one of claims 1 or 2, characterised in that with a multi-section valve body (4) there is arranged in the region of the fitting-in points (13) of the base body (1), intermediate element (2) and end element (3) in the continuous longitudinal bore (8) in the multisection valve body (4) an inner coating (15).

4. A seal for a male urethra according to one of claims 1 to 3, characterised in that the one-section valve body (5) and the end element (3) of the multi-section valve body (4) on the jacket of their respective rear ends have a rounded part (14).

5. A seal for a male urethra according to one of claims 1 to 4, characterised in that in the base body (1) of the valve body (4 and 5) on its front end there is arranged a cylindrical sleeve (16), open at both sides, in which as a seal (9) there is arranged an extractable plug, wherein the external edge of the sleeve (16) and the external edge of the seal (9) are flush with the front edge of the valve body (4 and 5).

6. A seal for a male urethra according to claim 1 characterised in that the external membrane (11) is formed either to be smooth or as concertina walls or a thin-walled bellows.

7. A seal for a male urethra according to one of claims 1 to 6, characterised in that the internal jacket membrane (10) has in the region of the radial bore (12) in the base body a reinforcing mushroom-shaped head (17).

**Revendications**

1. Obturation pour des conduits urinaires masculins, qui est formée d'un solide allongé qui doit être introduits dans le conduit urinaire et qui doit y être fixé, caractérisée en ce que le solide allongé est formé d'un corps de base (1) ou d'un corps de base (1) avec au moins un élément intermédiaire (2) et un élément terminal (3) et ainsi forme une valve cylindrique (4 et 5) ouverte des deux côtés ayant un ou plusieurs éléments pourvus d'un alésage longitudinal (8) d'un bout à l'autre, et dans lequel la valve (4 et 5) possède au moins à son extrémité antérieure une obturation (9) disposée séparable et dans laquelle l'enveloppe de la valve est partiellement entourée d'un tube à double paroi qui peut fonctionner par à-coups, en ce que le tube à double paroi est formé d'une paroi intérieure d'une enveloppe qui repose d'une manière glissante sur la valve et d'une paroi extérieure extensible (11), en ce que le corps de base (1) possède dans la zone antérieure au moins un alésage radial (12) et

**EP 0 301 292 B1**

en ce que la paroi intérieure de l'enveloppe possède un orifice de passage de l'air (18).

2. Obturation pour les conduits urinaires masculins selon la revendication 1, caractérisée en ce que dans un valve à plusieurs éléments (4), le corps de base (1) au moins un élément intermédiaire (2) et l'élément final (3) sont adaptés exactement et sont insérés mobiles d'une manière inséparable l'un dans l'autre et que le corps de base (1), l'élément intermédiaire (2) et l'élément terminal (3) possèdent le même diamètre intérieur et extérieur.

3. Obturation pour les conduits urinaires masculins selon l'une des revendications 1 ou 2, caractérisée en ce que pour une valve à plusieurs éléments (4) dans la zone des emplacements d'insertion (13) du corps de base (1), de l'élément intermédiaire et de l'élément terminal dans l'alésage longitudinal d'un bout à l'autre, un revêtement intérieur (15) est disposé dans la valve à plusieurs éléments (4).

4. Obturation pour des conduits urinaires masculins selon l'une des revendications 1 à 3, caractérisée en ce que la valve à un seul élément (5) et l'élément terminal (3) de la valve à plusieurs éléments (4) présentent sur l'enveloppe de leur extrémité postérieure correspondante, un arrondi (14).

5. Obturation pour des conduits urinaires masculins selon l'une des revendications 1 à 4, caractérisée en ce que l'on dispose dans le corps de base (1) de la valve (4 et 5) à l'extrémité antérieure de celui-ci une boîte cylindrique (16) ouverte des deux côtés, dans laquelle on dispose en tant qu'obturation un bouchon rétirable pour lequel les bords externes de la boîte (16) et les bords externes de l'obturation (9) sont montés serrés avec le bord antérieur de la valve (4 et 5).

6. Obturation pour des conduits urinaires humains selon la revendication 1, caractérisée en ce que la paroi externe (11) est développée soit sous forme lisse, soit comme une poche plissée ou comme une poche à lamelles.

7. Obturation pour des conduits urinaires masculins, caractérisée en ce que la paroi interne de l'enveloppe possède dans la zone du perçage radial (12) dans le corps de base, une tête de vis de renforcement.

Fig. 1

Fig. 2

EP 0 301 292 B1

fig. 4

fig. 5

fig. 3

10